# EUROPEAN PATENT SPECIFICATION

(11) **EP 3 359 010 B1**
(45) Date of publication and mention of the grant of the patent: **27.05.2026**
(21) Application number: 16854267.8
(22) Date of filing: 05.10.2016
(51) Int. Cl.: A61B 1/00, A61B 1/04, A61B 17/28, A61B 17/29, A61B 17/42, A61B 34/00

(54) **END-EFFECTOR JAW CLOSURE TRANSMISSION SYSTEMS FOR REMOTE ACCESS TOOLS**
ÜBERTRAGUNGSSYSTEME FÜR ENDEFFEKTOR-BACKENSCHLIESSUNG FÜR FERNZUGANGSWERKZEUGE
SYSTÈMES DE TRANSMISSION DE FERMETURE DE MÂCHOIRE D'EFFECTEUR D'EXTRÉMITÉ POUR OUTILS D'ACCÈS À DISTANCE

(30) Priority: 05.10.2015 US 201562237476 P; 05.10.2015 US 201562237483 P
(43) Date of publication of application: 15.08.2018
(73) Proprietor: LivsMed, Inc., Seongnam-si, Gyeonggi-do (KR)
(72) Inventor: ZIMMERMAN, Zachary, Brighton, MI 48116 (US); AWTAR, Shorya, Brighton, MI 48116 (US); JOHNSON, Bruce, Brighton, MI 48116 (US); HOLMES, Christopher, K., Brighton, MI 48116 (US); COSTA, Peter, F., Brighton, MI 48116 (US); RANK, Ryan Brook, Brighton, MI 48116 (US)
(74) Representative: J A Kemp LLP
(86) International application number: PCT/US2016/055606
(87) International publication number: WO 2017/062529

(56) References cited:
- WO-A2-2007/137304
- GB-A- 2 513 326
- US-A- 5 683 412
- US-A1- 2005 004 431
- US-A1- 2005 038 469
- US-A1- 2011 024 145
- US-A1- 2014 291 383
- US-A1- 2016 135 830
- US-B2- 8 992 422
- ANONYMOUS: "Six-bar linkage - Wikipedia", 11 July 2015 (2015-07-11), XP055583636, Retrieved from the Internet <URL:https://en.wikipedia.org/w/index.php?title=Six-bar_linkage&oldid=670945266> [retrieved on 20190426]

## Description

### FIELD

Described herein are transmission systems that are used for remote access instruments, for example minimally invasive surgical tools. In particular the apparatus provides a transmission system design that utilizes the transmission member as an energy storing device, over a certain portion of input stroke, to achieve a specific desired performance of the surgical tool. In general, the transmission member may be referred as jaw closure transmission member, or as jaw closure transmission cable, or as transmission cable, or as cable or the like.

### BACKGROUND

Typically, in laparoscopic, endoscopic, or other minimally invasive surgical procedure, a small incision or puncture is made in a patient's body. A cannula is then inserted into a body cavity through the incision, which provides a passageway for inserting various surgical devices such as scissors dissectors retractors or similar instruments. To facilitate operability through the cannula, instruments adapted for laparoscopic surgery typically embody a relatively narrow shaft supporting an end effector (EE) at its distal end and a lever or handle at its proximal end. Arranging the shaft of such an instrument through the cannula allows a surgeon to manipulate the proximal handle from outside the body to cause the distal end effector to carry out a surgical procedure at a remote internal surgical site. In most embodiments, the handle and tool shaft can be directly connected and roll rotation of the entire handle may drive rotation of the entire tool shaft and end-effector. Some alternative laparoscopic tools, such as, for example, US Patent No 8668702 exist where the handle is not directly connected to the tool shaft but via an input joint (e.g. comprising a pair of transmission strips) which still allow for roll rotation of the tool shaft and end-effector by way of handle rotation. In general, handle body may be referred as handle reference, or as palm grip, or the handle shell or the like.

A laparoscopic or endoscopic instrument may provide a surgeon with the ability to transfer high force loads from the proximal end of the tool to the distal end. These forces are transferred through the instrument through an Input, Output and Transmission Member Sub-System, where the sub-system consists of a mechanism, as seen in most surgical instruments such as US Patent No 5330502. The Input mechanism generally consists of an actuating lever body as an input and an output (for example, a shuttle coupled to the handle body via a 1 DoF slider joint). As a user actuates the handle lever, the motion is transferred to the shuttle, the amount that the shuttle displaces is based on the input mechanisms mechanical advantage, or transmission ratio. The terms transmission ratio and mechanical advantage are both used in this document since as the transmission ratio and mechanical advantage are, in general, simply the inverse of each other. When emphasizing force, the attribute mechanical advantage is used, and when emphasizing displacement, the attribute transmission ratio is used. Similarly, the output mechanism can have a varying mechanical advantage or transmission ratio over the output stroke. For a mechanical surgical instrument which requires a high force output while not compromising on output displacement, this varying mechanical advantage of both the input mechanism and the output mechanism have a certain desirable profile. The mechanical advantage at the initial segment of the stroke can be low because no force build up is required initially however the mechanical advantage at the end of the stroke needs to be high to allow a reasonable force input to be amplified into a large force output. The transmission members used in the prior art are generally stiff in the direction of transmission. However, this transmission member does not have to be rigid. In transmission system described here, the transmission member itself is designed to have a finite stiffness so that it acts as an energy storage member during certain portions of the input stroke of the device. This offers a unique performance of the device and has many benefits over rigid or highly stiff transmission members.

US 5 683 412 A discloses a force-limiting control member for an endoscopic instrument. The member is a flexible wire having a proximal and a distal end. A portion of the wire is bent into a sinusoidal or coiled shape to form a spring portion of the control member. An endoscopic instrument incorporating the force-limiting control member includes a flexible coil having a proximal and a distal end, a pair of forceps jaws mounted for rotation on a clevis which is coupled to the distal end of the coil, and a proximal handle coupled to the proximal end of the coil. The proximal handle has a spring biased lever coupled to it in a manner which limits the throw of the lever to a preselected distance. The force-limiting control member extends through the coil and is coupled at its distal end to the forceps jaws and at its proximal end to the actuating member. The spring portion of the control member which preferably resides inside the handle and is free to expand as the actuating member is moved from its spring biased first position to its throw limited second position. The spring portion of the control member is formed to have a spring constant such that when the actuation member is in its second throw limited position, the spring portion is expanded to a length which causes it to exert a predetermined force without exceeding its elastic limit. This is advantageously accomplished by pre-stressing the spring.

GB 2 513 326 A discloses an electrosurgical instrument for the treatment of tissue including an instrument shaft with an end effector comprising a pair of scissor blades capable of applying force to tissue located between the blades. The instrument includes at least first and second handle members, capable of relative movement towards and away from one another, and an actuator mechanism such that relative movement of the handle members causes the scissor blades to move between their open and closed positions. The actuator mechanism provides a first stage of operation in which the scissor blades are moved against one another to apply a first force to the tissue, the first force being limited to a predetermined threshold force. The actuator mechanism further provides a second stage of operation in which the scissor blades are moved against one another to apply a second force to the tissue, the second force being in excess of the predetermined threshold force.

US 2011/024145 A1 discloses a force-limiting clutch for a surgical instrument including a drive element and a transmission element. An actuator is coupled to the drive element, and a drive shaft is coupled to the transmission element for providing an output force. If the force input from the actuator to the clutch reaches a predetermined threshold, the force-limiting clutch "slips" and does not transfer the actuator force to the drive shaft to prevent excessive force/pressure output. Normally, drive features on the drive element engage with transmission features on the transmission element to transfer forces from the actuator to the drive shaft. When a threshold force is reached, the drive features slip past the transmission features. This clutch " breakpoint" determines the maximum loading that the surgical instrument can provide at its output.

### SUMMARY OF THE DISCLOSURE

According to the present invention there is provided the medical device of claim 1. Additional aspects of the invention are set out in the dependent claims. Described herein are jaw closure transmission systems that provide enhanced closure security and feel. These closure transmissions may be part of any appropriate apparatus, including medical devices (e.g., minimally invasive surgical tools), or any other application in which it is beneficial or desirable to have a jaw closure mechanism that may securely grip and provide feedback to the user on grip strength, as will be described herein.

In general, the jaw closure transmission systems described herein may include rigid and compliant transmission elements, including an input (e.g., a jaw actuation input), an output (e.g., jaw mechanism), a transmission cable having a finite stiffness in a transmission direction, and rigid or flexible transmission guiding element, wherein the transmission element stores energy during closure transmission to achieve unique and desirable functionality.

The jaw closure transmission systems described herein may include three (or more) sub-systems that are serially connected that take an input, in the form of handle lever displacement and force from the user, and produce an output that presents as moving jaw displacement and clamp load. In general, moving jaw may be referred as movable jaw, or as moveable jaw or as end-effector moving jaw, or as EE moving jaw or the like. The three sub-systems are as follows: (a) Input Sub-system: Handle Assembly; (b) Output Sub-system: Jaw Assembly; (c) Transmission Sub-system (e.g., transmission member, e.g. Cable, and transmission guide, e.g. Flexible Conduit). The Input Sub-system may include the input in a Handle Assembly that comprises a handle body or shell that serves as the local reference or ground, and a handle lever configured to receive user input in the form of closing or displacing the handle lever relative to the handle body. In general, the full closure displacement of the handle lever with respect to the handle body is referred to as the input stroke. At full closure, i.e. at the end of input stroke, the handle lever reaches a hard-stop relative to the handle body. At this hard stop, there may be a single locking or latching feature that keeps the handle lever latched closed relative to the handle body. An unlatching/unlocking feature (e.g., a releasable lock) unlocks the handle lever and allows it to open again with respect to the handle body.

The handle assembly may also include a handle output (handle mechanism output) that connects to the transmission cable. The handle output may be a shuttle, a push rod, pull rod, etc. The output typically interfaces with the transmission member and provides an actuation motion to the proximal end of the Transmission Member.

In general, the handle mechanism is configured as a mechanical linkage system that translates the closing motion of the handle lever relative to the handle body to a corresponding actuation motion of the handle shuttle relative to the handle body. The handle mechanism provides a transmission ratio and mechanical advantage between the handle lever and the handle shuttle so as to produce the appropriate actuation displacement and force at the proximal end of the transmission member (e.g., appropriate cable tension and cable displacement) via the handle shuttle during the overall stroke of the handle lever (i.e., input stroke). This optimization may be based on the structure and functionality of the overall jaw closure transmission system including the input sub-system, transmission sub-system, and output sub-system, and in some variations may not be due to just the input sub-system.

The handle mechanism may be designed such that instead of providing a constant mechanical advantage or transmission ratio, it produces a higher transmission ratio (e.g., lower mechanical advantage) in the first portion of the input stroke and a lower transmission ratio (higher mechanical advantage) in the second portion of the input stroke.

The output sub-system typically includes an end-effector assembly or jaw assembly, and may include the following elements: an end-effector (e.g., jaw) base or end-effector fixed jaw that serves as the local reference or ground; an end-effector movable jaw coupled to the end-effector fixed jaw (e.g., pivotally coupled to the end-effector fixed jaw) such that it can open and close (i.e. displace) with respect the end-effector fixed jaw.

The full closure displacement of the moving jaw relative to the fixed jaw may be referred to as the output stroke; in the devices described herein, the output stroke is always complete prior to completion of the input stroke, and is generally completed around the transition between the first portion of the input stroke and the second portion of the input stroke (e.g., between about 30% and 70% of the full input stroke, e.g., between about 40% and 60% of the full input stroke, between about 40% and 70% of the full input stroke, between about 45% and 60% of the full input stroke, etc.).

In general, once the jaws have been closed either against themselves or against an object grasped in the jaws, the jaws of the jaw assembly are at a stop position, and will no longer close further (full output stroke), by the action of the handle assembly actuating the transmission cable. However, because the transmission cable has a finite stiffness in a transmission direction (e.g., is somewhat compliant), the handle assembly may continue to be actuated, in the second part of the input stroke, and may stretch the transmission cable. This stretch may be felt by the user operating the handle (as resistance in the handle) and the force being applied to stretch the cable may be transmitted to the jaws as a holding force between the jaws.

Thus, a closure displacement of the Handle Lever relative to the Handle Body at the input of the Closure Transmission System may result in a closure displacement of the moving jaw relative to the fixed jaw to hold an object (such as needle, suture, tissue, staple, clip etc.) between the jaws.

A pulley coupled to the fixed jaw (e.g. pivotally coupled to the fixed jaw) may be configured to receive the actuation motion from the distal end of the transmission member. The jaw assembly may include a jaw mechanism, which may be a linkage, cam (e.g., cam surface and pin), etc. The jaw mechanism (e.g. in some variations a drive pin / cam surface) that translates the actuation motion of the jaw pulley relative to the fixed jaw to a corresponding closure motion of the moving jaw relative to the fixed jaw.

In one example of the jaw mechanism, a drive pin is driven by the pulley and interfaces with a camming surface on the moving jaw, providing a camming action. In one example of the jaw mechanism, the distal end of the transmission member (i.e. cable) is wrapped around the pulley pulley. To prevent a potential slippage between the cable and the pulley, there is a positive engagement feature between the cable and the pulley. This accomplished via a cylindrical member that is crimped onto the cable and sits in a cavity on the pulley. The jaw mechanism is designed to provide a transmission ratio and mechanical advantage between the distal end of the transmission member and the moving jaw so as to produce the appropriate output displacement and force at the moving jaw relative to the fixed jaw during the overall stroke of the moving jaw (i.e. output stroke). This optimization may be based on the structure and functionality of the overall closure transmission system including the input sub-system, transmission sub-system, and output sub-system, and not just the output sub-system. Specifically, the jaw assembly (end-effector) mechanism may be designed to provide a large mechanical advantage at the end of its stroke, to maximally amplify the force in the transmission member (i.e., tension in the jaw closure transmission cable) to a clamping force at the jaws. This implies that for a certain desired jaw clamping force, the transmission cable tension can be less, which has several advantages.

The transmission sub-system may include a transmission member to transmit the closure action of the input sub-system (i.e. handle assembly) to the output sub-system (i.e. jaw assembly) of the closure transmission system. More specifically, the transmission member may transmit the actuation motion of the handle shuttle to a corresponding actuation motion of the jaw pulley. This transmission member may be a cable, braided rope, etc. that is capable of accommodating very tight bends as might necessary when the closure transmission system is part of a remote access tool or device.

The transmission member may be highly compliant (i.e. flexible) in bending, twisting, and compression. This member is relatively stiffer in tension because it has to transmit force and displacement along this direction; but at the same time it is not chosen or designed to be infinitely or effectively infinitely stiff. Rather, it is intentionally designed or chosen to have a finite stiffness (or finite compliance) so that it can also serve as an inline spring. In general, nothing is infinitely stiff or infinitely compliant; infinite stiffness corresponds to zero compliance and zero stiffness corresponds to infinite compliance. Instead, stiffness may be scaled on a relative scale. For example, on some normalized scales a stiffness less than 10 is close to infinitely compliant and a stiffness greater than 1000 is closely to infinitely stiff. In any of the apparatuses described the axial stiffness of the transmission member may have a stiffness in the range of 100 may be used.

Any of these apparatuses may include a transmission guide that serves as a conduit or channel (also, a reference) for the Transmission Member. This proximal portion of this transmission guide is connected to the Input Sub-system reference (i.e. Handle Body) and the distal portion of this transmission guide is connected to the Output Sub-system reference (i.e. EE Fixed Jaw). This guide may be completely rigid in all directions such as a frame or a shaft or tube. Alternatively, this guide may be flexible in bending so that it can take an arbitrary tortuous shape but still remains very stiff (ideally, close to infinitely stiffness) axially (i.e. along its bent/deformed central axis). This guide may be flexible in bending so that it can take an arbitrary tortuous shape sand have an intermediate stiffness (i.e., have some intentionally finite compliance) in the axial direction (i.e., along its bent/deformed central axis).

The connections between the ends of the guide and respective references of the input and output sub-systems maybe close to infinitely stiff in the transmission direction (i.e. axial direction of the transmission cable) or may have some intentionally finite compliance (i.e. slightly lower stiffness than infinitely stiff values).

Collectively, the three coupled sub-systems may allow for the use of cables as the primary transmission member. Cables are highly flexible in bending and therefore can be incorporated within minimal access tools / devices that have an input articulation joint between the handle and the tool frame/shaft, and an output articulation joint between the tool frame/shaft end-effector. In such devices, the tool frame/shaft may also serve as a portion of or the entire transmission guide. In particular, the use of cable a transmission member enables a very tight bend at the output articulation joint. This helps facilitate the miniaturization of output articulating joint, and therefore the end effectors as well at the distal end of the minimal access tool / device.

Furthermore, the choice of a cable as a transmission member and a flexible conduit as a transmission guide member facilitates a minimal access tool/device architecture where the handle assembly is not directly connected to a tool frame/shaft. Instead, in some variations of the devices described herein, the handle assembly "floats" with respect to the tool shaft/frame, and may be connected via a virtual center input articulating joint that is proximal to the handle assembly. The system (apparatus) may include either or both a flexible conduit as the transmission guide member to guide the transmission member (cable) from the handle assembly to the tool shaft/frame.

Furthermore, the choice of a cable as the jaw closure transmission member in an articulating minimal access tool/device may also ensure a relative decoupling between the jaw closure functionality of the device and articulation functionality of the device. Since the transmission member itself does not have significant bending (i.e. articulation) stiffness, it does not significantly impact the articulation of the end-effector (jaw assembly). Assembly about the output articulation joint. Moreover, a large mechanical advantage in the jaw mechanism may result in a lower or limited tension in the transmission cable, which has several advantages listed in Point 3 below. Also, lower tension in jaw closure transmission cable reduces jumpiness (lateral jerk due to lateral movement of the high tension jaw closure transmission cable) and S-bending (distortion of the joint due to buckling along its center axis) in the output articulating joint (refer the EE articulating joint patent application).

This overall Jaw Closure Transmission System may enable jaw closure in two steps. During the first portion of the input stroke, as the handle lever moves from its fully open position to an approximately mid-way open position (typically about 30%-70% of the stroke), the moving jaw goes from it fully open position to its fully closed position. In this state, the jaw mechanism has achieved its full output stroke and has reached a hard-stop. This hard-stop may be the result of jaw on jaw contact, or the two jaws holding a needle in between. In either case, the jaw mechanism has reached a static state, while there is still input stroke remaining at the handle mechanism. This point onwards, the remaining stroke of the handle mechanism goes into axially stretching the transmission member (i.e. cable) and or axially compressing the transmission guide members. The intentional axial compliance selected in the transmission member and transmission guide member (discussed above) enables the user to continue to displace the Handle lever through the remaining portion (i.e. the second portion) of the Handle Mechanism's overall Input Stroke. During this second portion of the Input Stroke, the actuation motion of the Handle Shuttle causes the transmission member (e.g. cable) to stretch and/or Transmission Guide Member (e.g. flexible conduit) to compress since the distal end of the transmission member is static due to the static state of the jaw mechanism. Thus the second portion of the Input Stroke corresponds to stretching the cable and an associated increase in tension of the cable (based on the compliance of the cable). This gradually increasing cable tension continues to serve as the input force on the jaw mechanism and continues to get amplified by the mechanical advantage of this mechanism (even though the mechanism itself is static due to a hard-stop at the jaws). This means, that the clamping force between the jaws (with or without a needle in between) keeps increasing as well. Thus, while the first portion of the input stroke of the handle lever corresponds to an increasing displacement of the moving jaw from a fully open position to a fully closed position (i.e. total Output Stroke), which corresponds to a hard-stop at the jaws (with or without a needle); the second portion of the Input Stroke at the Handle Lever corresponds to a gradually increasing clamping force between the jaws (with or without a needle) at the EE Assembly. The embodiment may not only be present as a two-stage stroke, but also may be present as a three-stage stroke wherein the third stage relates to a region dedicated to facilitating Handle Lever locking. Within the third stage the Handle Lever angular displacement does not produce additional Transmission Member displacement, and therefore does not introduce any additional energy to the compliant transmission elements. The primary purpose of the third stage is to provide a single region where the Handle Mechanism locks into place. The presence of this third stage provides for an opportunity to optimize Handle Mechanism design for locking, rather than for facilitating Jaw Mechanism closure or clamp load generation. Specifically, the input force required throughout the third stage does not depend on the mechanical spring-rate property for the compliant transmission members, but rather merely depend on frictional losses between the members. As a result of isolating Transmission Sub-System and Output Sub-System kinematic behavior from the third stage, users of the device will experience a greater consistency of Handle Mechanism input force. Furthermore, the user input force in the third stage to lock the handle is significantly independent of needle location within the jaws of the Output Mechanism.

This may result in several benefits for the user and in the design, including the substantial reduction in sudden step changes in the force feedback felt by the surgeon at the Handle Lever as needle contact or jaw contact happens. The presence of transmission member and/or transmission guide member compliance in the axial (i.e. transmission motion) direction makes this transition more gradual and therefore better in feel for the user, compared to a traditional device that has a highly rigid transmission member. This may also result in a reduced number of transmission elements since energy storage functionality is accomplished through the dual-purpose cable and flexible guide members, which play a role in actuation motion transmission as well as serve as energy storing elastic elements. During the second portion of the Input Stroke of the Handle Lever, the transmission sub-system efficiently stores energy by means of stretching the transmission cable. This energy storage is not passive, in the sense that the stretching of the cable corresponds to an increase in cable tension, which when reflected through the mechanical advantage of the EE mechanism produces an increased jaw clamping force.

In general, the jaw closure transmission systems described herein may be a self-limiting and/or self-correcting and/or self-regulating system for limiting the maximum force that is transmitted via the transmission member in spite of variations in the presence and location of a an object (e.g., needle) in the jaws. This may advantageously lower the loads all members/components of the Jaw Closure Transmission System. This may also or alternatively lead to less wear, longer life, less chances of failure, more durability etc., and may eliminate the need for complex input, transmission, and output force overload systems that might require additional springs, linkages, and structural members. In addition, these jaw closure transmission systems described herein may regulate needle clamp load which helps reduce damage to needles, and/or may desensitize the system from size and location of needle held between the jaws. Provides an adequate clamping force without damaging the needle. These jaw closure transmission systems may also regulate handle lever force applied by the surgeon which is preferred from an ergonomic standpoint. In the case of a rigid transmission member, it becomes very difficult for the surgeon to regulate the clamping force at the jaws by adjusting his input force/displacement at the handle lever. In such cases, a very small change in the surgeon's input displacement at the Handle Lever can produce a large, somewhat uncontrolled, change in the clamping force. That is why in such systems, there are discrete ratchet points between the Handle Body and Handle Lever that allows the surgeon to incrementally increase the clamping force at the jaws in controlled amounts. The present Jaw Closure Transmission System with the intentional use of compliance in the transmission member and transmission guide members provides the surgeon with a much greater control of the clamping force at the jaws, thus mitigating or eliminating the need for discrete ratchets at the Handle Lever (with respect to Handle Body). Rather, this system lets the surgeon rely upon his feel and discretion to regulate input force to achieve a desired needle clamping force. This also eliminates the need for complex multi-lock ratcheting mechanism in the handle assembly. Removing a multi-ratchet system de-features the handle. Wherein a ratcheting system requires an additional user actuation component/input to disengage the locking mechanism. If this feature wasn't included in the multi-ratchet system, the tool would have to overload the needle or object in the jaws to release it. The handle becomes simpler with a single lock design as it does not require an additional user input to disengage the lock. Simplifying the handle reduces potential user error and could result in less user training.

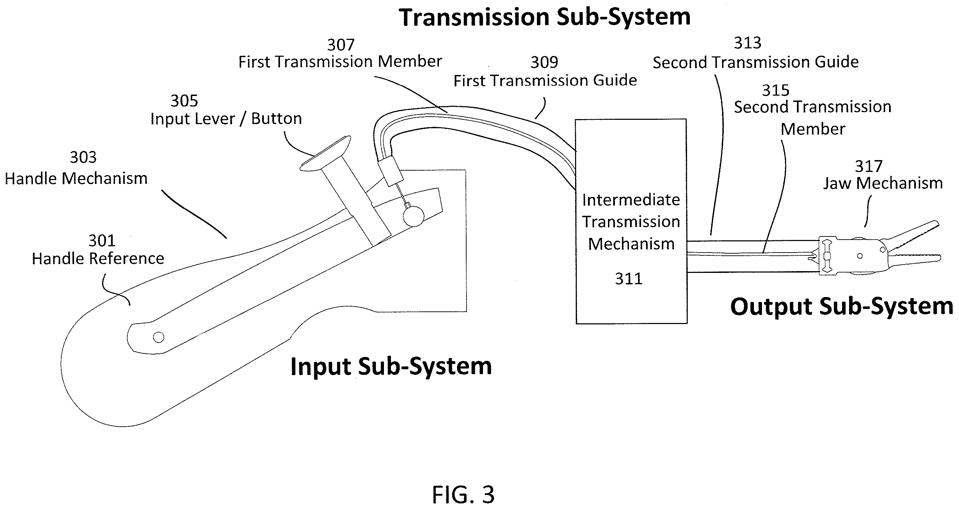

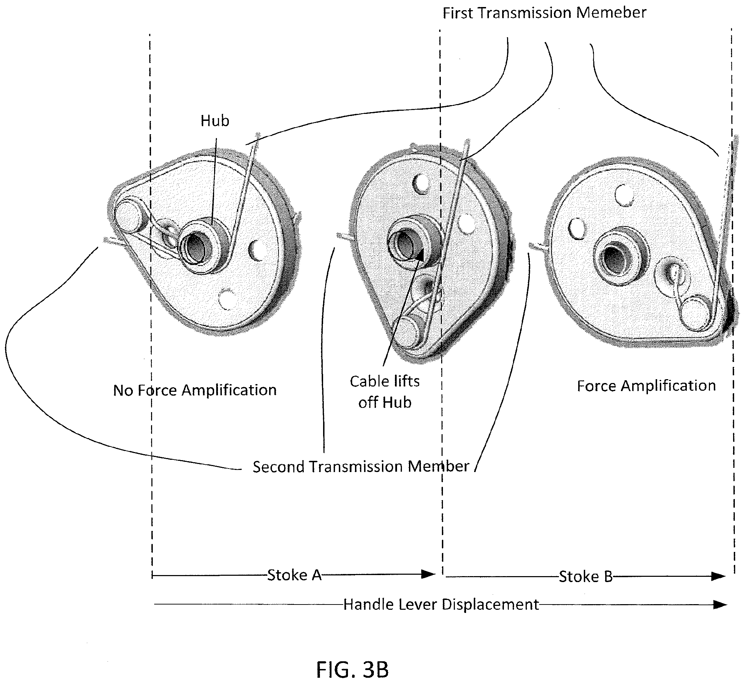

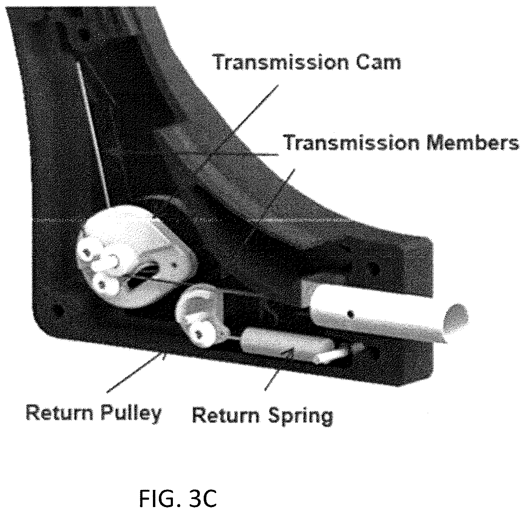

Also described herein are jaw closure transmission systems in which an additional intermediate sub-system may be used. FIG. 3 illustrates one example of such a system, showing a handle reference 301, handle mechanism 303, input lever/button 305, first transmission member 307, first transmission guide 309, intermediate transmission mechanism 311, second transmission guide, 313, second transmission member 315, and jaw mechanism 317. For example, in addition to an Input Sub-system and an Output Sub-system, there may also be an Intermediate Sub-System with an Intermediate Mechanism. In that case there may be a first Transmission Member and Transmission Guide Member between the Input and Intermediate Sub-Systems, and a second Transmission Member and Transmission Guide Member between the Intermediate and Output Sub-Systems. The use of an axially compliant transmission and transmission guide members may be preserved to achieve desired Jaw Closure performance.

In any of the apparatuses described herein the handle input may be a lever, or any other input allowing a variable degree of actuation, which may generally be referred to herein as "levers", including plungers, dials, knobs, etc.

As mentioned, these jaw closure transmission systems may generally provide for connecting an input and an output comprising rigid and compliant transmission elements, as well as rigid and flexible transmission guiding elements, wherein the transmission elements with finite flexibility in the transmission direction also serves to store energy during closure transmission to achieve unique and desirable functionality.

Thus, in a simple form, the system can be thought of as, but not limited to, three sub-systems that are serially connected that take an input, in the form of handle lever displacement by force from the user, and produce an output that presents as moving jaw displacement and clamp load.

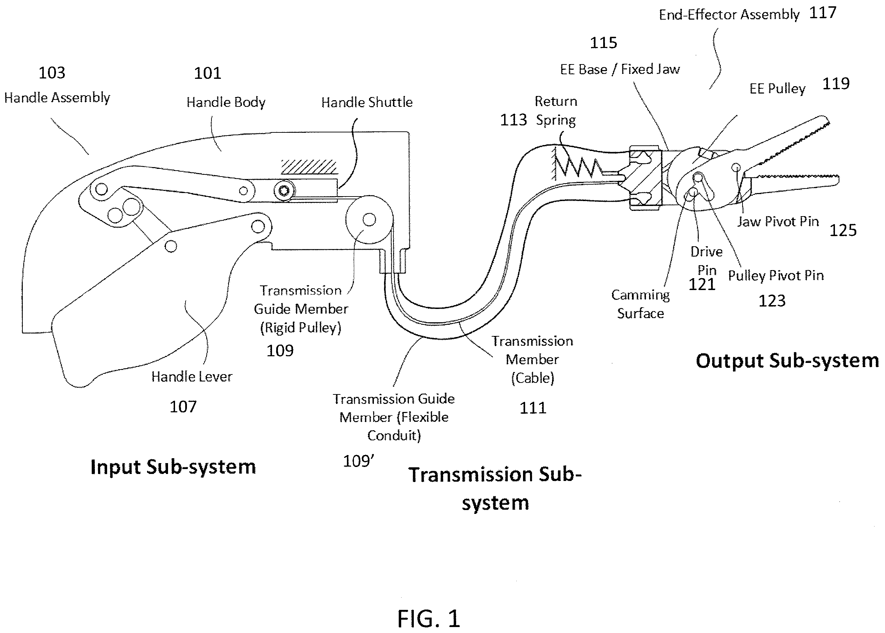

The three sub-systems (Input Sub-system which is referred to as the Handle Mechanism; Output Sub-system which is referred to as the Jaw Mechanism; and Transmission Sub-system which comprises of a Transmission Member, e.g. Cable, and Transmission Guide, e.g. Flexible Conduit) may be represented in a system diagram as shown in one example in FIG.1. The example shown in FIG. 1 includes a handle body or handle shell 101, a handle assembly 103 comprising a handle mechanism, a handle lever 107 (input lever or input link), a transmission guide 109 (riding pulley) and/or flexible conduit 109' a transmission cable 111, a return spring 113, a fixed jaw 115 (end effector base/reference) an end-effector assembly 117 comprising a jaw mechanism, a drive pin 121, a pulley pivot pin 123, a pulley 119, and a jaw pivot pin 125.

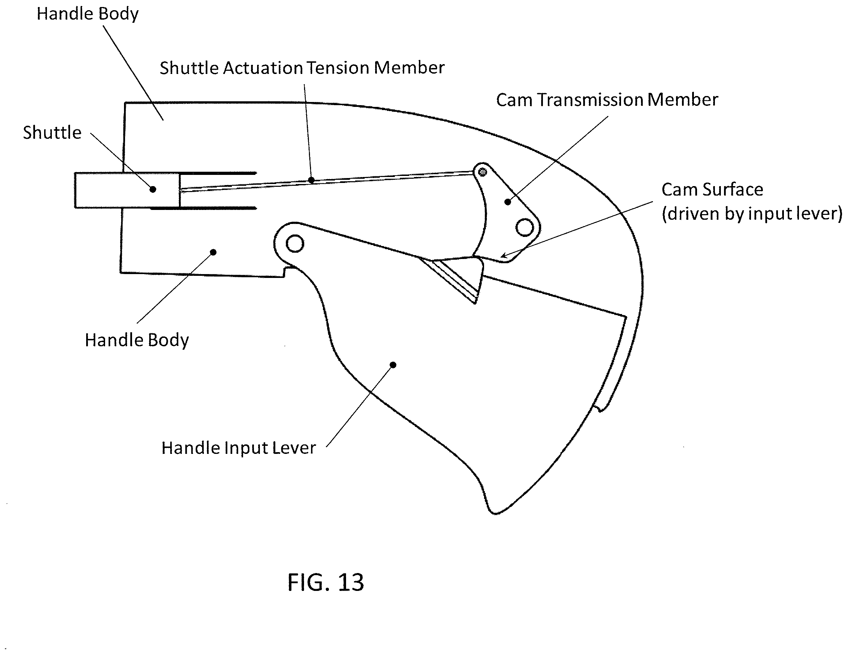

The input in a Handle Assembly may comprise a Handle Body or Shell that serves as the local reference or ground. The handle body is generally designed to be ergonomic for the user to hold in various positions since it is generally the articulation of the handle body which controls the location and orientation of the end effector. Mechanically the handle body can be directly connected to the end effector via a tool shaft as in straight stick laparoscopic instruments, serially or connected to the end effector thought through an input articulating joint, a tool frame (e.g. a frame, or a frame with a shaft extension, or a shaft), and an output articulating joint a series of joints which provide articulation to the end effector or even indirectly attached to the end effector as described in US Patent No 8668702. The Handle body houses an internal mechanism (or handle mechanism) consisting of a Handle Lever configured to receive user input in the form displacement relative to the Handle Body. Full Handle Lever displacement with respect to the handle body is referred to as the Input Stroke. This input stroke is based on the kinematic design of the handle mechanism and is limited by one more hard-stops in the handle mechanism. This input stroke is designed to have a specific mechanical advantage curve profile that, when combined with the other sub assembles, is unique to the type of surgical instrument. Generally, for a surgical needle driver, the mechanical advantage curve of the input sub-system initially comprises of a low mechanical advantage then increases to have a high mechanical advantage at the end of the input stroke. At full closure, i.e. the end of Input Stroke, the Handle Lever reaches a hard-stop relative to the Handle Body. At this hard stop, there may be a single locking or latching feature that keeps the Handle Lever latched closed relative to the Handle body. As mentioned above an unlatching/unlocking feature may unlock the Handle Lever and allow it to open again with respect to the Handle Body. The output of the Handle Mechanism is via the Handle Shuttle (or output member, pull rod, or push rod), which interfaces with the Transmission Member and provides an actuation motion to the proximal end of the Transmission Member. The output of the Handle Mechanism is not limited to a Shuttle, the embodiment shown consist of a "Shuttle" because the Handle Mechanism is a 6 bar linkage with a 1 DoF slider joint between the output member (shuttle) and handle body. The handle mechanism is not limited to a 6 bar linkage. The handle mechanism could be a simple lever, 4 bar linkage, cam slot, gear, etc. The Handle Mechanism may be configured to provide a varying transmission ratio and mechanical advantage between the Handle Lever and the Handle Shuttle so as to produce the appropriate actuation displacement and force at the proximal end of the Transmission Member (i.e. appropriate cable tension and cable displacement) via the Handle Shuttle during the overall stroke of the Handle Lever (i.e. Input Stroke). The Handle Mechanism itself may be take the form of various configurations. As opposed to a six-bar linkage as cited, in some variations, the linkage system may be a 4-bar linkage, or any alternate system containing a plurality of linkages or motion members that actuates the transmission member either by rotary of linear motion. Conversely, any of the linkages contained within the linkage system could be driven by a cam purposefully designed to induce a variable mechanical advantage throughout the handle's jaw closure lever stroke. Fig. 13 shows an Input Sub-System consisting of a cam which achieves the desired variable mechanical advantage. In some variations, the linkage system may be a compliant mechanism that achieves the desired constant or variable transmission ratio. This mechanism may lead to part count reduction by still achieving similar performance. In some variations, the Handle Mechanism is designed such that instead of providing a constant mechanical advantage or transmission ratio, it is designed to produce a higher transmission ratio (lower mechanical advantage) in the first portion of the Input Stroke and a lower transmission ratio (higher mechanical advantage) in the second portion of the Input Stroke. While the mechanism for input into the Input Sub-System generally includes an actuating lever body, or ground reference, and a Handle Input Lever, the Input Sub-System may be embodied alternatively. The Input Sub-System may be embodied as a motion member capable of translating mechanical energy therein. For example, the input motion member may be a button, dial, tension rod, or binary switch.

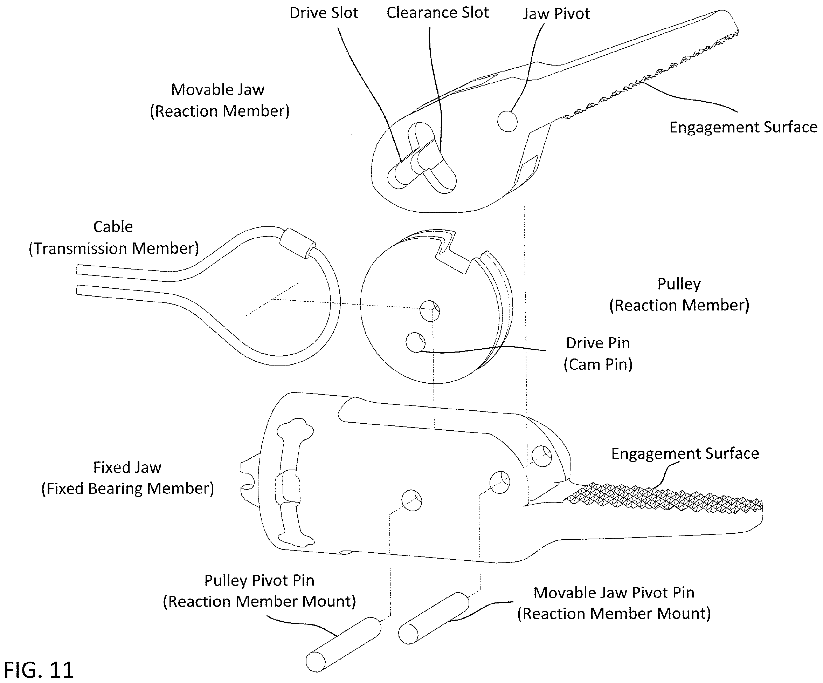

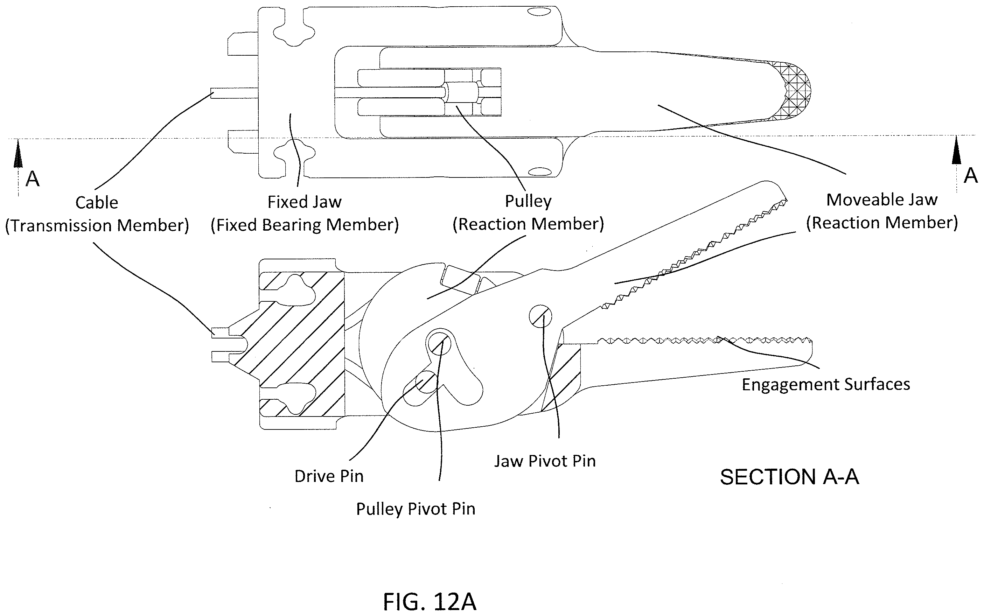

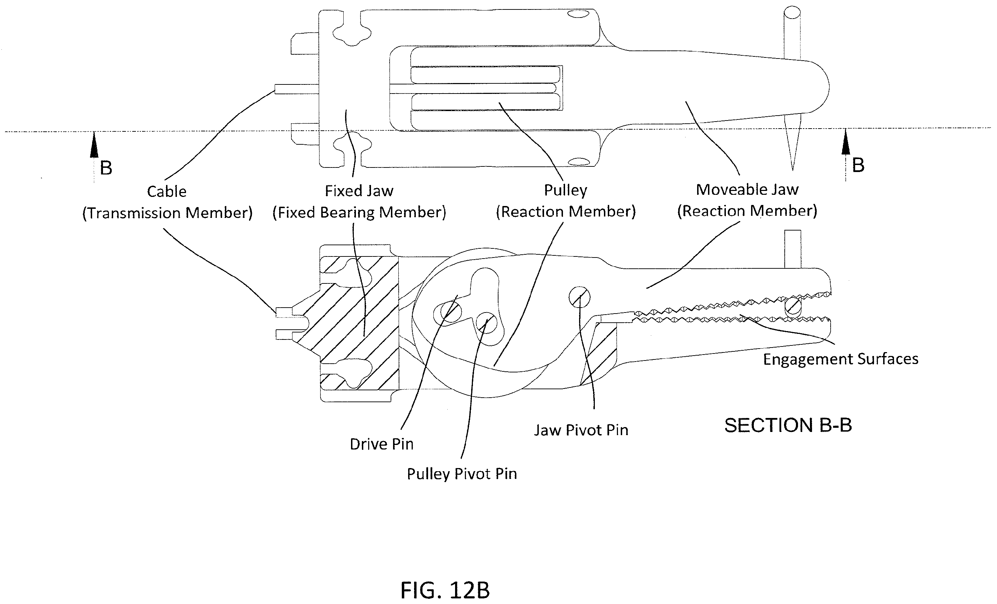

The Output may generally be a Jaw Mechanism comprising a jaw base (which may include or be integral with a) fixed Jaw that serves as the local reference or ground (alternatively two moving jaws may be used), and the movable Jaw may be coupled to the Fixed Jaw (e.g., pivotally coupled to the Fixed Jaw) such that it can open and close (i.e. displace) with respect the Fixed Jaw. The structure of one end-effector (jaw assembly) embodiment is seen FIGS. 1, 2, 11, 12A, and 12B. As discussed above, the full closure displacement of the End Effector Moving Jaw relative to the End Effector Fixed Jaw is referred to as the Output Stroke. The purpose of this closure displacement of the End Effector Moving Jaw relative to the End Effector Fixed Jaw is to hold an object (such as needle, suture, tissue, staple, clip etc.) between the jaws in response to a corresponding closure displacement of the Handle Lever relative to the Handle Body at the input of the Closure Transmission System. The embodiment shown incorporates but is not limited to, a two stage mechanical mechanism to produce the desired mechanical advantage curve. The desired mechanical advantage curve being low mechanical advantage to start with and then high mechanical advantage at the end. The design is not limited to the current embodiment as long as the mechanical advantage curve is conserved. In the embodiment shown an End Effector Pulley coupled to the End Effector Fixed Jaw or the fixed bearing member (e.g. pivotally coupled to the End Effector fixed jaw) configured to receive the actuation motion from the distal end of the Transmission Member. A Jaw Mechanism (e.g. comprising a drive pin / cam surface) that translates the actuation motion of the end effector Pulley relative to the Fixed Jaw to a corresponding closure motion of the Moving Jaw relative to the end effector Fixed Jaw. In the example of the end effector Mechanism, a Drive pin is driven by the end effector Pulley and interfaces with a camming surface on the Moving Jaw, providing a camming action. Additionally, in the example of the End Effector Mechanism, the distal end of the Transmission Member (i.e. cable) is wrapped around the End Effector Pulley. To prevent a potential slippage between the cable and the pulley, there may be a positive engagement feature between the cable and the pulley. This may be accomplished via a cylindrical member that is crimped onto the cable and sits in a cavity on the pulley. Once the cable is wrapped around the pulley, it is connected to a Return Spring either in the jaw assembly, or on the transmission guide, or in the handle assembly. The purpose of this Return Spring is to open the jaws after fully closure is reached and the Handle Lever returns to the initial open angle. The End Effector mechanism is designed to provide a varying Transmission Ratio and Mechanical Advantage between the distal end of the Transmission Member and the End Effector Moving Jaw so as to produce the appropriate output displacement and force at the end effector Moving Jaw relative to the end effector Fixed Jaw during the overall stroke of the end effector Moving Jaw (i.e. Output Stroke). This optimization is based on the structure and functionality of the overall Closure Transmission System including the Input Sub-system, Transmission Sub-system, and Output Sub-System, and not just the Output Sub-system. Specifically, the end effector mechanism is designed to provide a large mechanical advantage at the end of its stroke, to maximally amplify the force in the Transmission Member (i.e. tension in the jaw closure transmission cable) to a clamping force at the jaws. This implies that for a certain desired jaw clamping force, if the Mechanical Advantage of the jaw mechanism is high when the jaws are closed, the Transmission Cable tension can be lower, which has several advantages. The end-effector may include many different embodiments but is not limited to a pair of jaws, useful for manipulation of needles, suture, tissue, cautery, ligation clip application, etc.

The Transmission Sub-system may comprise the following elements, a Transmission Member to transmit the closure action of the Input Sub-system (i.e. Handle Assembly) to the Output Sub-System (i.e. end effector Assembly) of the Closure Transmission System. More specifically, the Transmission Member transmits the actuation motion of the Handle Shuttle to a corresponding actuation motion of the end effector Pulley. More specifically, this Transmission Member is a cable, braided rope, etc. that is capable of accommodating very tight bends as might necessary when the Closure Transmission System is part of a Remote Access Tool or Device as seen in FIGS. 1, 10, 15 and 16. The transmission member is highly compliant (i.e. flexible) in bending, twisting, and compression. This member is relatively stiffer in tension because it has to transmit force and displacement along this direction; but at the same time it is not chosen or designed to be infinitely or effectively infinitely stiff. Rather, it is intentionally designed or chosen to have a finite stiffness (or finite compliance) so that it can also serve as an inline spring. The importance of this finite stiffness for the system level performance is described below. Note that that nothing is infinitely stiff or infinitely compliant. Infinite stiffness corresponds to zero compliance and zero stiffness corresponds to infinite compliance. On some normalized scale, a stiffness less than 10 is close to infinitely compliant and a stiffness greater than 1000 is closely to infinitely stiff. On such a scale, a stiffness in the range of 100-200 is where we might place the axial stiffness of the transmission member.

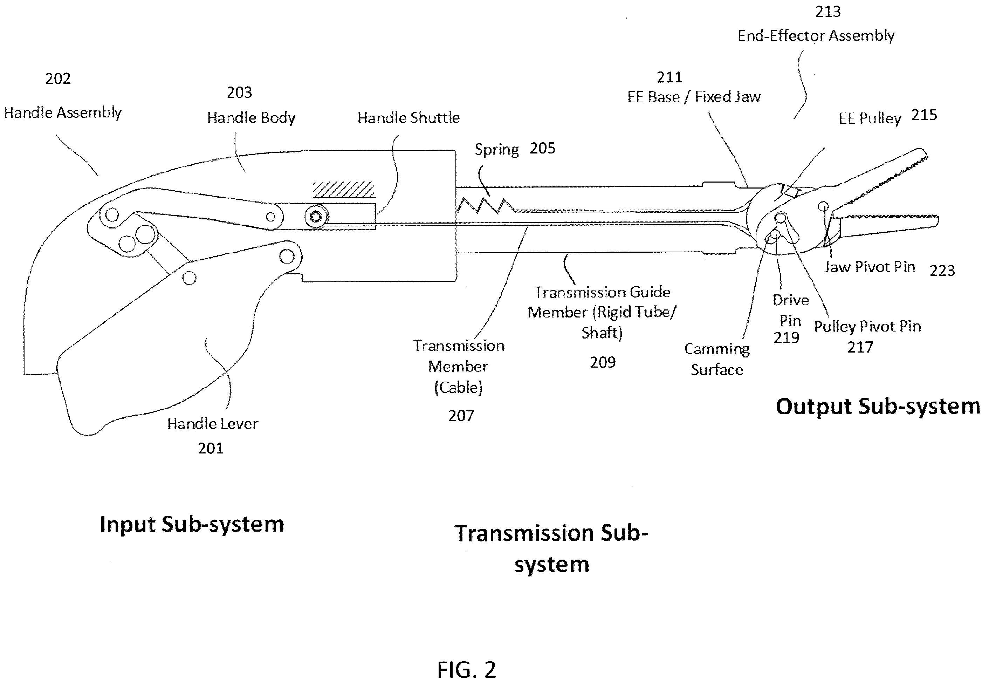

A Transmission Guide that serves as a conduit or channel (also, a reference) for the Transmission Member. This proximal portion of this transmission guide is connected to the Input Sub-system reference (i.e. Handle Body) and the distal portion of this transmission guide is connected to the Output Sub-system reference (i.e. end effector Fixed Jaw). This guide may be completely rigid in all directions such as a frame or a shaft or tube as seen in Fig.2. This guide may also be flexible in bending so that it can take an arbitrary tortuous shape but still remains very stiff (ideally, close to infinitely stiffness) axially (i.e. along its bent/deformed central axis). Additionally, this guide may be flexible in bending so that it can take an arbitrary tortuous shape and have an intermediate stiffness (i.e. have some intentionally finite compliance) in the axial direction (i.e. along its bent/deformed central axis). The connections between the ends of the guide and respective references of the input and output sub-systems maybe close to infinitely stiff in the transmission direction (i.e. axial direction of the transmission cable) or may have some intentionally finite compliance (i.e. slightly lower stiffness than infinitely stiff values). FIG. 2 shows a handle assembly 202 comprising a handle mechanism, a handle lever 201 (input link or input lever), a cable 207, a body body (or handle reference) 203, a return spring 205, a guide member 209, a fixed jaw 211 (end effector base or reference), an end effector assembly 213 comprising a jaw mechanism, a pulley 215, a pulley pivot pin 217, a drive pin 219, and a jaw pivot pin 223.

For example, describe herein are medical devices having a jaw assembly actuated by a transmission cable having a finite stiffness in a transmission direction. For example the devices may include: an elongate transmission guide, wherein the transmission cable is routed through the transmission guide; a handle assembly at a proximal end of the elongate transmission guide, the handle assembly comprising a handle body, an input lever, a handle output coupled to the transmission cable, and a handle mechanism coupling the input lever to the handle output, wherein the handle mechanism has an input stroke consisting of a full closure displacement of the input lever relative to the handle body, further wherein the input stroke is divided into a first part and a second part, wherein the first part corresponds to a displacement of 30% to 70% of the full closure displacement of the input lever and the second part corresponds to the remaining displacement of the input lever; and wherein the jaw assembly is distal to the elongate transmission guide, the jaw assembly having a first jaw, a second jaw, a jaw input coupled to the transmission cable, and a jaw mechanism coupling the jaw input to the second jaw, wherein the jaw mechanism has an open configuration when the first and second jaws are fully open relative to each other and a closed configuration when the first and second jaws are fully closed; further wherein the displacement of the input lever relative to the handle body corresponding to the first part of the input stroke actuates the handle output which in turn actuates the jaw input via the transmission cable, which in turn closes the first and second jaws until the first and second jaws reach a hard stop, and thereafter the displacement of the handle lever relative to the handle body corresponding to the second part of the input stroke stretches the transmission cable, wherein the resulting tension in the transmission cable is converted by the jaw mechanism to a holding force between the first and second jaws.

A medical device having a jaw assembly actuated by a transmission cable having a finite stiffness in a transmission direction may include: an elongate transmission guide comprising a flexible conduit, wherein the transmission cable is routed through the transmission guide; a handle assembly at a proximal end of the elongate transmission guide, the handle assembly comprising a handle body, an input lever, a handle output comprising a shuttle coupled to the transmission cable, and a handle mechanism comprising a six bar linkage coupling the input lever to the handle output, wherein the handle mechanism has an input stroke consisting of a full closure displacement of the input lever relative to the handle body, further wherein the input stroke is divided into a first part and a second part, wherein the first part corresponds to a displacement of 30% to 70% of the full closure displacement of the input lever and the second part corresponds to the remaining displacement of the input lever; and wherein the jaw assembly is distal to the elongate transmission guide, the jaw assembly having a first jaw, a second jaw, a jaw input comprising a pulley coupled to the transmission cable, and a jaw mechanism comprising a cam surface between the jaw input and the second jaw, wherein the jaw mechanism has an open configuration when the first and second jaws are fully open relative to each other and a closed configuration when the first and second jaws are fully closed; further wherein the displacement of the input lever relative to the handle body corresponding to the first part of the input stroke actuates the handle output which in turn actuates the jaw input via the transmission cable, which in turn closes the first and second jaws until the first and second jaws reach a hard stop, and thereafter the displacement of the handle lever relative to the handle body corresponding to the second part of the input stroke stretches the transmission cable, wherein the resulting tension in the transmission cable is converted by the jaw mechanism to a holding force between the first and second jaws.

The handle mechanism may be a linkage (e.g., six-bar linkage, four-bar linkage, etc.) or a cam (cam surface and pin, etc.). In general, the elongate transmission guide may comprise a flexible conduit or elongate shaft or both.

The transmission cable may generally have a finites stiffness in the direction of transmission (e.g., along the length of the extended cable). For example the transmission cable may have a stiffness in a transmission direction of less than 800 pounds per inch, less than 700 pounds per inch, less than 650 pounds per inch, less than 600 pounds per inch, less than 500 pounds per inch, less than 400 pounds per inch, etc. (and in some variations be greater than 100 pounds per inch, greater than 150 pounds per inch, greater than 200 pounds per inch, greater than 250 pounds per inch, greater than 300 pounds per inch, etc., e.g., between 100 and 650 pounds per inch, etc.). One pound per inch is equivalent to 6895 Pa.

In any of these apparatuses (devices, systems, mechanism, tools, etc.) the handle mechanism may be configured to provide a first mechanical advantage during the first part of the input stroke and a second mechanical advantage that is greater than the first mechanical advantage during the second part of the input stroke. The handle output may comprise one or more of: a shuttle, a push rod, or a pull rod. The device may include a jaw base to which either or both the first and second jaws are pivotally coupled. The jaw input may comprise a jaw pulley, and the jaw mechanism may comprise a cam surface between the jaw pulley and the second jaw.

As mentioned above, any of these devices may include a releasable latching mechanism configured to hold the handle lever locked in a closed position at the end of the input stroke.

Also described herein are methods of using any of the apparatuses including these jaw closure transmission systems, which are illustrated as examples for understanding the workings of the device only and have not been claimed. For example, described herein are methods of operating a medical device to close a jaw assembly of the medical device, wherein the medical device comprises an elongate transmission guide, a finite stiffness transmission cable within the transmission guide, and a handle assembly at the proximal end of the elongate transmission guide having an input lever and a handle mechanism coupling the input lever to the transmission cable, wherein the transmission cable is coupled to a jaw input of the jaw assembly, wherein the jaw assembly is distal to the elongate transmission guide. The method may include: actuating the input lever to apply tension to the transmission cable during a first part of an input stroke of the handle assembly to close a first and second jaw of the jaw assembly from an open configuration until the first and second jaws reach a hard stop; and continuing to actuate the input lever during a second part of the input stroke after the first and second jaws have reached the hard stop and stretching the transmission cable; wherein the input stroke consists of a full displacement of the handle lever of the handle assembly, and further wherein the handle assembly transitions from the first part of the input stroke to the second part of the input stroke when the handle is between 30% and 70% displaced.

A method of operating a medical device to close a jaw assembly of the medical device, wherein the medical device comprises an elongate transmission guide, a finite stiffness transmission cable within the transmission guide, and a handle assembly at the proximal end of the elongate transmission guide having an input lever and a handle mechanism coupling the input lever to the transmission cable, wherein the transmission cable is coupled to a jaw input of the jaw assembly, wherein the jaw assembly is distal to the elongate transmission guide, may include: actuating the input lever to actuate the transmission cable during a first part of an input stroke of the handle assembly and translate the transmission cable relative to the elongate shaft to close a first and second jaw of the jaw assembly from an open configuration until the first and second jaws reach a hard stop; and continuing to actuate the input lever and stretching the transmission cable without translating the first or second jaws during a second part of the input stroke after the first and second jaws have reached the hard stop; wherein the input stroke consists of a full displacement of the handle lever of the handle assembly, and further wherein the handle assembly transitions from the first part of the input stroke to the second part of the input stroke when the handle is between 30% and 70% displaced.

Any of these methods may include applying a first mechanical advantage during the first part of the input stroke and applying a second mechanical advantage that is greater than the first mechanical advantage during the second part of the input stroke. These methods may also include grasping an object between the first and second jaws, wherein the first and second jaws reach the hard stop when the object is secured between the first and second jaws.

Any of these methods may also include locking the input lever in a fully closed position relative to a handle shell in the handle assembly.

Any of these methods may also include releasing the input lever to transition the handle lever from the second part of the input stroke to the first part of the input stroke, reducing the tension on the transmission cable and reducing the stretch of the transmission cable before translating the transmission cable so that the first and second jaws open. Actuating the input lever may comprise squeezing the input lever.

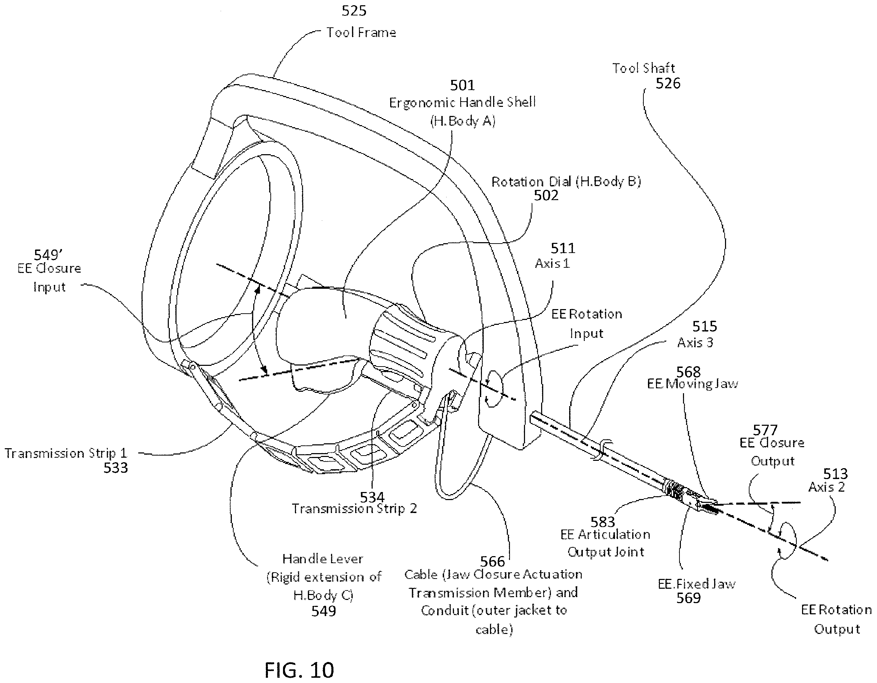

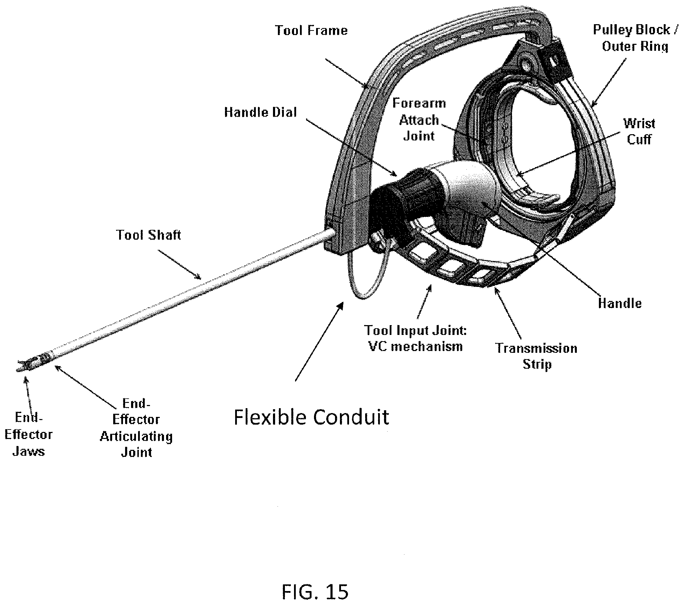

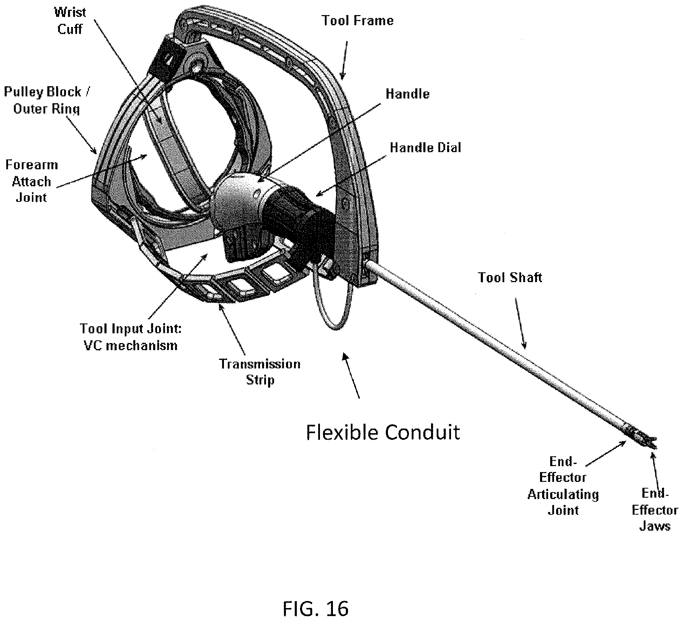

As mentioned, these jaw closure transmission systems may be integrated into any appropriate apparatus. For example, any of these apparatus may be configured as a medical device, see for example FIGS. 15 and 16, including a jaw closure transmission system. For example, a medical device having a distal jaw assembly actuated by a transmission cable having a finite stiffness in the transmission direction and is compliant in bending may include: a tool frame comprising an elongate shaft and a forearm attachment region at a proximal end of the tool frame configured to couple with an arm attachment cuff; a handle assembly, the handle assembly comprising a handle shell configured to be gripped in a user's palm and an input lever on the handle shell, wherein the handle shell encloses a handle linkage coupling the input lever to the transmission cable through a handle output, further wherein the handle assembly has an input stroke consisting of a full closure displacement of the input lever from an undisplaced configuration to a fully displaced configuration, further wherein the input lever transitions from a first part of the input stroke to a second part of the input stroke when the input lever is displaced from an undisplaced configuration to between 30% and 70% of its full closure displacement configuration; an input joint between the handle and the tool frame configured to encode motion of the handle about a pitch axis of rotation relative to the tool frame for transmission to an articulating output joint, and further configured to encode motion of the handle about a yaw axis of rotation relative to the tool frame for transmission to the articulating output joint, wherein the pitch axis of rotation and the yaw axis of rotation intersect in a center of rotation; wherein the jaw assembly is coupled to the distal end of the elongate tool shaft by the articulating output joint, the jaw assembly having a first jaw, a jaw pulley pivotally coupled to the first jaw and further coupled to the transmission cable, a second jaw pivotally coupled to the first jaw, and a cam surface that translates motion of the jaw pulley to a motion of the second jaw relative to the first jaw, wherein the jaw assembly has an output stroke that extends from an open configuration when the first and second jaws are fully open to a closed configuration when the first and second jaws are fully closed; wherein the displacement of the input lever relative to the handle body corresponding to the first part of the input stroke actuates the handle output which in turn actuates the jaw input via the transmission cable, which in turn closes the first and second jaws until the first and second jaws reach a hard stop, and thereafter the displacement of the handle lever relative to the handle body corresponding to the second part of the input stroke stretches the transmission cable, wherein the resulting tension in the transmission cable is converted by the jaw mechanism to a holding force between the first and second jaws; and a transmission guide extending between the handle assembly and the elongate shaft, wherein the transmission cable extends from the handle assembly, through the transmission guide to the jaw assembly. FIGS. 10, 14, 15, and 16 illustrate one example of such an apparatus.

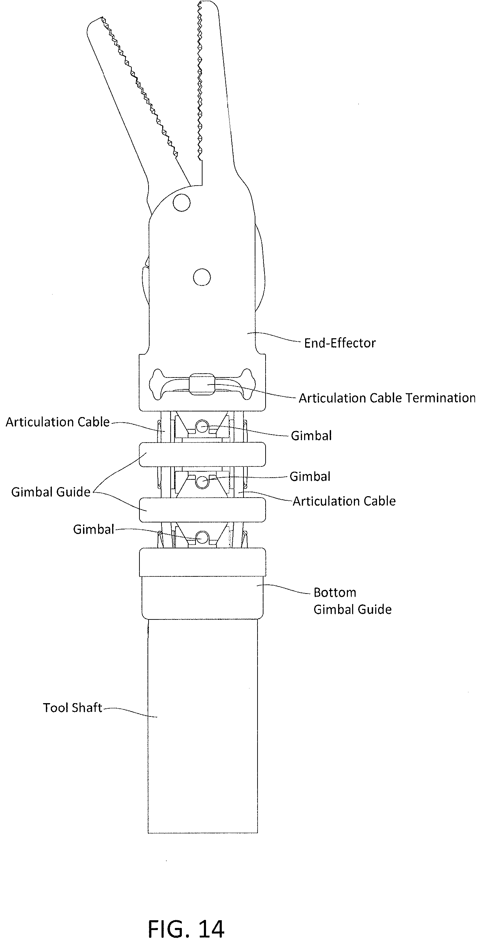

In FIG. 10, a medical device apparatus includes a jaw closure transmission as described above. The exemplary apparatus includes a tool frame 525, which includes a tool shaft 526 and a forearm attachment portion at the proximal end 527. A cuff (not shown) having a passage therethrough that is configured to hold a wrist or forearm of a user may be coupled to the forearm attachment portion; in some variations via a bearing between the forearm attachment portion of the frame and the cuff that is configured to slide or roll so that there is a roll rotational degree of freedom between the frame and the cuff about the tool axis. A proximal handle assembly may be connected to the tool frame by an input joint. The input joint may be configured to encode motion between the tool frame and the handle assembly, as shown in FIG. 10. In this example, the input joint includes a pair of transmission strips 533, 534 that connect to respective pivoting joints (not shown) in parallel to separately encode pitch and yaw rotations of the handle assembly. The output joint 583 (an end-effector articulation joint configured as a jaw assembly), as shown in FIG. 14, may be any of the multi-cluster joints described herein and is between the jaw assembly and the tool frame (e.g., tool shaft) receives transmission input (e.g., cables, not shown) from the output joint 533, 534 to articulate the jaw assembly.

In this example, the handle assembly includes an ergonomic palm grip portion 501 (handle shell) that connects to the rotation dial 502. The handle assembly also includes a control (lever) 549 input (in this example, defining the end-effector jaw closure input 549) that is configured as a handle lever and acts as a rigid extension of the internal push rod. A transmission cable 566 connects to the shuttle and acts as a jaw closure actuation transmission member extending from the shuttle and through the tool shaft to the jaw assembly. This transmission cable may be enclosed by a protective and/or supporting sheath or cover or conduit, for some or entire portion of its length. The end-effector is a jaw assembly including a first (ground) end-effector portion, in this example, including a fixed jaw 569 to which a pivoting second end-effector portion (moving jaw 568) is attached. The transmission cable 566 may couple to the moving jaw at the end-effector closure output 577.

In FIG. 10, rotation of the dial portion of the handle assembly when the user's forearm is mounted to the proximal end and the palm grip region is held in the user's hand so that the user can rotate the dial between the thumb and fingers, rotates the entire tool frame, and therefore the end-effector that is attached to the distal end of the tool frame via an end-effector output articulating joint. Thus, the handle may rotate about first axis 511 referred to as handle articulated roll axis (axis 1), to cause the tool shaft to rotate in a third axis 515 referred to as the tool shaft roll axis (axis 3), in turn causing the end-effector to roll about a second axis, referred to as an end-effector articulated roll axis (axis 2).

The rotation dial 502 as shown in FIG. 10 is rotated about axis 1 511. The rotation leads to rotation of tool frame 525 via transmission strips 533, 534 (as they constrain rotation DoF), tool shaft 526 (about axis 3 515) and therefore, the end-effector (about axis 2 513). When handle is articulated using the input articulating joint, the output joint (multi-cluster joint 583) and end-effector articulates via the output articulating joint. Now, the center axis (axis 2) for end-effector is different from the axis 3, the shaft axis. The Intermediate Transmission Mechanism consist of but not limited to a cam mechanism that is seen in as seen in Fig. 3B. The Intermediate Transmission Mechanism could be a linkage, gear, cog etc. During Stroke A, the force is not being amplified through the Intermediate Transmission mechanism however at the transition from Stroke A to Stroke B the First Transmission Member jumps lifts off the Hub and rides a surface farther away from the cams center of ration, creating a force amplification from the First Transmission Member to the Second Transmission Member. This force amplification increases the mechanical advantage of the system. This mechanism is shown in the structure of the device in Fig. 3C. While this Transmission System invention is specifically embodied as a laparoscopic, endoscopic, or other minimally invasive surgical jaw closure device, it is understood that those skilled in the art can alternately translate the invention, without departing from the scope of the claims, to alternate embodiments for Transmission Systems such as those that require end-effector clamping action like grasping, holding, or clamping instruments.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 shows an example of a system diagram for an example of a jaw closure transmission system consisting of an Input Sub-System, a Flexible Transmission Sub-System and an Output Sub-System.
FIG. 2 is another example of a diagram showing an example of a jaw closure transmission system consisting of an Input Sub-System, a Rigid Transmission Sub-System and an Output Sub-System.
FIG. 3 is another example of a diagram showing an example of a jaw closure transmission systems with an intermediate transmission mechanism incorporated into the jaw closure transmission systems. Fig. 3B shows a physical embodiment of Intermediate Transmission Cam used to create a force amplification from the First Transmission Member to the Second Transmission Member. Fig. 3C shows a physical embodiment of Intermediate Transmission Mechanism located inside a device/ tool.
FIG. 4 shows various needle cross sections that are commonly used in minimally invasive surgery and may be grasped by an apparatus including any of the jaw closure transmission systems described herein.
FIG. 5 illustrates various needle geometries that are commonly used in minimally invasive surgery.
FIG. 6 shows various needle sizes that are commonly used in minimally invasive surgery.
FIG. 7 is a front view of Needle Driver Jaws Clamping down on a curved needle.
FIG. 8 shows graphs illustrating the Transmission System of a Needle Driver's Input Stroke.
FIG. 9 shows a mechanical advantage profile for the entire system as a function of the input handle lever displacement.
FIG. 10 illustrates one example of a medical device incorporating a jaw closure transmission system as described herein.
Fig. 11 shows an exploded view of the end effector assembly.
Fig. 12A shows a detailed view of end effector assembly where the moveable jaw is in open condition.
Fig. 12B shows a detailed view of end effector assembly where the moveable jaw is grasping a needle.
Fig. 13 shows an input sub-system comprising a cam in the handle mechanism.
Fig. 14 shows an embodiment of end-effector assembly including the output articulation joint.
Fig. 15 show embodiment of a minimally invasive surgical device that incorporates jaw closure transmission system described here.
Fig. 16 shows embodiments of a minimally invasive surgical device that incorporates jaw closure transmission system described here.

### DETAILED DESCRIPTION

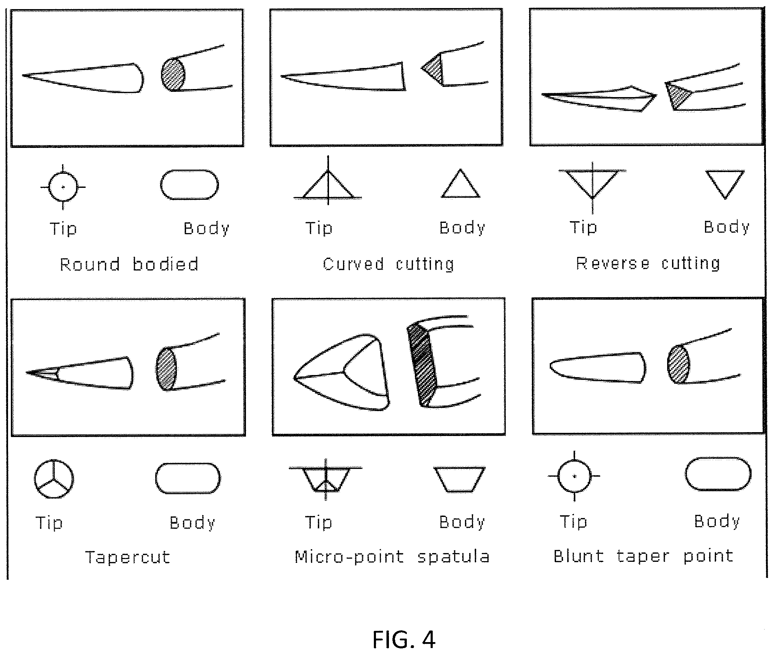

Described herein are jaw closure transmission systems and apparatuses including them. For example, described herein are transmission system (jaw closure transmission systems) for a remote access tool which incorporates a transmission member with finite transmission direction stiffness (or equivalently, a compliant transmission member) that interfaces with the input and output mechanisms. A relatively stiff transmission member of a previous medical device can't be replaced with a compliant transmission member and achieve the performance described herein. The transmission system in its entirety must be designed in unison to achieve the performance that will be described. The performance of the transmission system in its current configuration is specific for a needle driver. Surgical needle drivers are one hand operation devices which require high clamping loads at jaw clamping surfaces in order to drive various needles through tissues. It is important to understand the various types of needles because the design of a compliant transmission member can protect the needle from damage when overdriving the jaws. Fig.4 has various needle types that are selected based on the medium that they are driven through. The body of the needle is just an important as the tip in that as the needle is driven through the tissue there is an interaction between the needle in its entirety and the tissue. The jaws of a needle driver are designed with a pattern intended to increase the needle retention without requiring high jaw clamping loads. However, if a large enough clamping load is applied to the needle the clamping surfaces will damage the needle body, leaving permanent impressions on the need surface. When the surface of the needle is damaged it will no longer slide smoothly through the tissues which will result in resistance to surgeon and unnecessary damage to the patient.

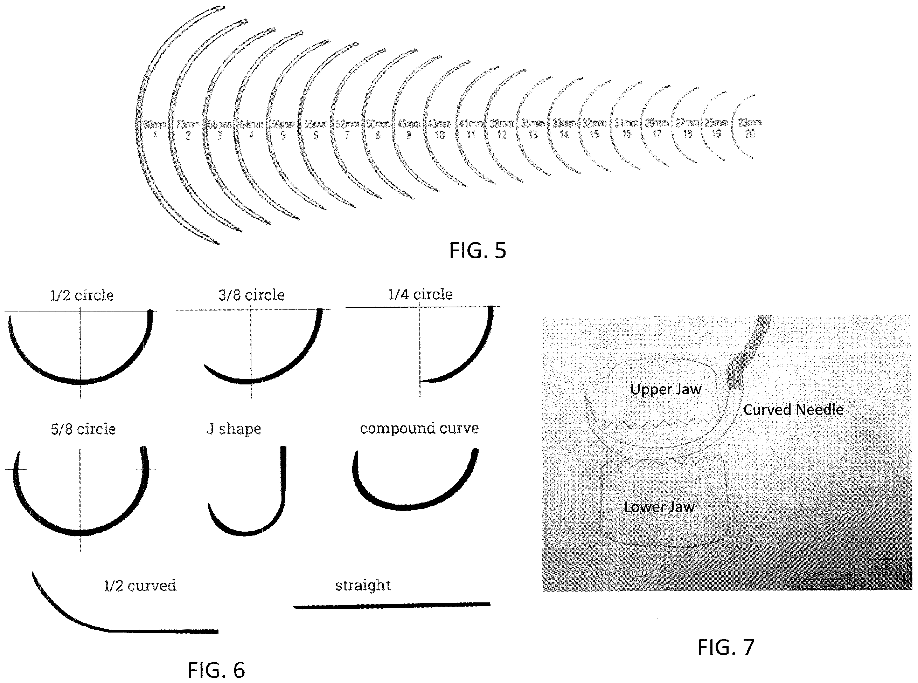

In addition, to surface damage on the needle an overload in clamping force could cause the shape of the needle to permanently deform. Needles used for minimally invasive surgery also come in various shapes and sizes seen in Fig.5 and Fig.6 respectively. The Jaws of the needle driver are designed to be wide enough to not allow the needle to rotate as a result needles with a larger curve and smaller diameter can be easily deformed and straighten out by large clamping loaded. Fig.7 showed a curved needle being held by the upper and lower jaws of a needle driver and as a larger clamping load is applied, the needle would straighten in that region due to three-point bending, causing the needle to not drive through the tissue in a true arc.

The needle location in the jaws also influences the corresponding jaw clamping force and impacts ability to adequately secure a needle. The needle can be placed anywhere along the jaw length which could mean at the very tip of the jaws or at the mouth of the jaws, this significantly changes the effort required by the user to actuate the input mechanism completely to full stroke. In some configurations full stroke should not be achieved due to potential damaging the needle therefore typical needle drivers incorporate input ratcheting system, where the stroke of the handle can be broken up into finite segments in between ratchets to allow the user to hold the needle at various input lever locations.

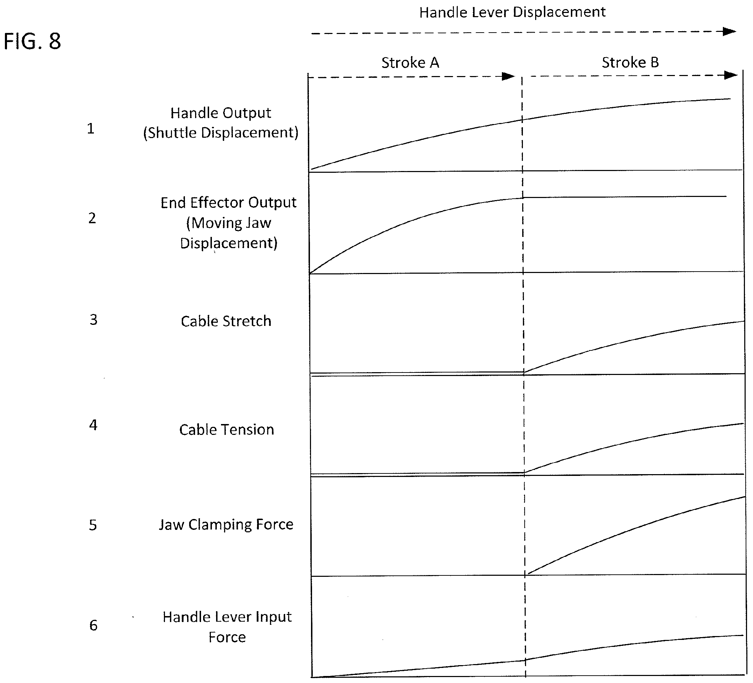

The use of a compliant transmission member solves these problems and eliminates the discrepancy among uses on amount of input to adequately hold the needle. A compliant transmission member acts as an energy storage member so that the user can actuate the input handle lever completely with having to worry about over driving the jaws and damaging the needle. If a large needle is placed within the mouth of the jaws and full stroke can still be achieved at the handle input lever while in a needle driver with a stiff transmission member full stroke would not be achievable without causing damage to the instrument or the needle. This reduces the need for a multiple ratchet system which can provide discrepancies to users on whether adequate jaw clamping force is achieved. The Handle Lever Displacement (Input Stroke) can be broken up into two different phases, Stoke A and Stroke B. Whereas the transition from Stroke A to Stroke B occur when the jaws reach a hard stop such that Stroke A is pre jaw hard stop and Stroke B being post Jaw hard stop. Jaw hard stop could occur at various handle lever displacements depending on the needle type, needle location or even needle presence. Fig.8 below shows various graphs that help explain what happens in the system as a fully input stroke is achieved.

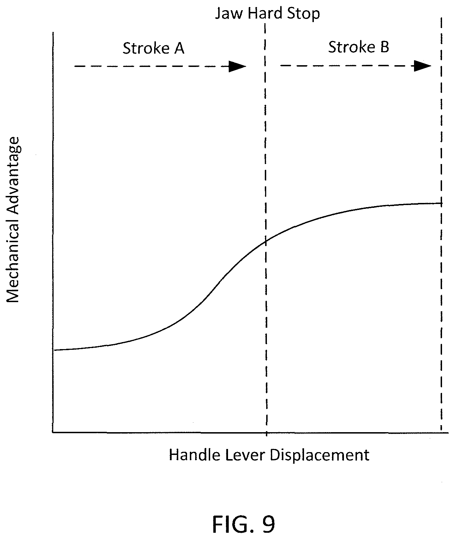

FIG. 8 Graph 1 shows the handle output, the profile of this curve is achieved through the geometry of the handle mechanism. This graph indirectly shows the mechanical advantage and the transmission ratio of the handle mechanism. This profile is extremely important as it is a non-constant mechanical advantage which consist of a low mechanical advantage at the beginning of the input stroke and then increases the mechanical advantage towards the end of the stroke. Due to ergonomic reasons (or limits), the limit of input displacement and force at the handle varies throughout the lever stroke (through the range of angular displacement). A varying mechanical advantage in the system means that during Stroke A can have a completely different transmission ratio than in Stroke B. During Stroke A, the jaws are freely rotating in space therefore a high transmission ratio and low mechanical advantage can be implemented into the design during this phase which enables the jaws to achieve a wide opening angle. While in Stroke B, when the jaws reach a hard stop, a higher mechanical advantage is desired such that a large clamping load at the output can ergonomically be applied from the input. The system transmission ratio comes from two sources, the handle mechanism and the jaw mechanism. The jaw mechanism which is seen in FIG. 8 graph 2 has a similar mechanical advantage and transmission ratio curve as the handle mechanism, low mechanical advantage to start then high mechanical to end the stroke. However, the jaw mechanism has a different stroke than the input mechanism. The entirely of the jaw mechanism stroke is contained within Stroke A because the transition between Stroke A and Stroke B happens when the jaws reach a hard stop and the mechanism thereon remains fixed providing a constant mechanical advantage for the rest of the input stroke. The profile of the systems net mechanical advantage curve is seen in FIG. 9. This profile allows the user to apply a large clamping load at very little effort at the handle which still achieving a large jaw opening angle.

Graph 3 in FIG. 8 shows the performance of the compliant transmission member (cable). Since there is no force build up in Stroke A the cable does not stretch however in Stroke B the cable is no being stretched. The cable is stretched because the jaw mechanism is fixed at the distal end while the input handle mechanism is still able to produce more cable displacement as the handle input lever reaches a full displacement (full stroke). A system with a much stiffer transmission member such a steel rod or a flexible control wire will not perform in this manner as displacement at the input handle would be really hard to generate because the forces would directly relate to the clamping forces on the needle. A compliant transmission member allows for a soft buildup of force at the handle over a displacement to generate the closure force required. During Stroke A the force felt at the handle input is the handle return spring which is shown in Fig.8 to have a linear spring constant K. As the Stroke transitions to Stroke B the handle force is now the sum of the handle return spring and then tension in the cable, the more compliant the transmission member the less drastic the increase of input force is at the Stroke A to B transition. As the handle input lever achieves full stroke the needle clamping force increases greatly based on the compliance of the cable and the amount of handle lever displacement left in Stroke B. A Transmission member that is too compliant would mean inadequate clamping load while a Transmission member that is too stiff would require a ratcheting system and could damage the needle. Even as the needle clamping force increases the handle lever input force doesn't increase as much due to the increasing high mechanical advantage of the handle mechanism and the fixed mechanical advantage of the jaw mechanism. This gradual increase in handle force while achieving optimal jaw closure eliminated the need of the multiple ratchet system that is required on needle drivers with a really stiff transmission member.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be conjointly employed in the methods and articles (e.g., compositions and apparatuses including device and methods). For example, the term "comprising" will be understood to imply the inclusion of any stated elements or steps but not the exclusion of any other elements or steps.

In general, any of the apparatuses and methods described herein should be understood to be inclusive, but all or a subset of the components and/or steps may alternatively be exclusive, and may be expressed as "consisting of" or alternatively "consisting essentially of" the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/-2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims. The examples and illustrations included herein show, by way of illustration and not of limitation, specific embodiments in which the subject matter may be practiced. As mentioned, other embodiments may be utilized and derived therefrom, such that structural and logical substitutions and changes may be made without departing from the scope of this disclosure. Thus, although specific embodiments have been illustrated and described herein, any arrangement calculated to achieve the same purpose may be substituted for the specific embodiments shown. This disclosure is intended to cover any and all adaptations or variations of various embodiments.

## Claims

1. A medical device having a jaw assembly (117, 213, 317) actuated by a transmission cable (111, 207, 307, 566) having a finite stiffness in a transmission direction that is highly compliant in bending, and stiffer in tension to transmit force in the transmission direction, the device comprising:
an elongate transmission guide (109', 209, 309), wherein the transmission cable (111, 207, 307, 566) is routed through the transmission guide (109', 209, 309);
a handle assembly (103, 202, 303) at a proximal end of the elongate transmission guide (109', 209, 309), the handle assembly (103, 202, 303) comprising a handle body (101, 203, 301, 501), an input lever (107, 201, 305, 549), a handle output coupled to the transmission cable (111, 207, 307, 566), and a handle mechanism (303) coupling the input lever (107, 201, 305, 549) to the handle output, wherein the handle mechanism (303) has an input stroke consisting of a full closure displacement of the input lever (107, 201, 305, 549) relative to the handle body(101, 203, 301, 501), further wherein the input stroke is divided into a first part and a second part, wherein the first part corresponds to a full closure of the jaw assembly (117, 213, 317) with a displacement of 30% to 70% of the full closure displacement of the input lever (107, 201, 305, 549) and the second part corresponds to the remaining displacement of the input lever (107, 201, 305, 549); and
wherein the jaw assembly (117, 213, 317) is distal to the elongate transmission guide (109', 209, 309), the jaw assembly (117, 213) having a first jaw (569, 115, 211), a second jaw (568), a jaw input coupled to the transmission cable (111, 207, 307, 566), and a jaw mechanism (317) coupling the jaw input to the second jaw (569, 115, 211), wherein the jaw mechanism (317) has an open configuration when the first (569, 115, 211) and second (568) jaws are fully open relative to each other and a closed configuration when the first (569, 115, 211) and second (568) jaws are fully closed;
further wherein the displacement of the input lever (107, 201, 305, 549) relative to the handle body (101, 203, 301, 501) corresponding to the first part of the input stroke actuates the handle output which in turn actuates the jaw input via the transmission cable (111, 207, 307, 566), which in turn closes the first (569, 115, 211) and second (568) jaws until the first (569, 115, 211) and second (568) jaws reach a hard stop, and thereafter the displacement of the input lever (107, 201, 305, 549) relative to the handle body (101, 203, 301, 501) corresponding to the second part of the input stroke stretches the transmission cable (111, 207, 307, 566), wherein a resulting tension in the transmission cable (111, 207, 307, 566) is amplified by a camming surface of the jaw mechanism (317) to a holding force between the first (569, 115, 211) and second (568) jaws.

2. The device of claim 1, wherein the handle mechanism (303) comprises a linkage or a cam.

3. The device of claim 1, wherein the handle mechanism (303) comprises a six-bar linkage.

4. The device of claim 1, wherein the elongate transmission guide (109', 209, 309) comprises a flexible conduit or elongate shaft or both.

5. The device of claim 1, wherein the transmission cable (111, 207, 307, 566) has a stiffness in a transmission direction of less than 4.48 MPa (650 pounds per inch).

6. The device of claim 1, wherein handle mechanism (303) is configured to provide a first mechanical advantage during the first part of the input stroke and a second mechanical advantage that is greater than the first mechanical advantage during the second part of the input stroke.

7. The device of claim 1, wherein the handle output comprises one or more of: a shuttle, a push rod, or a pull rod.

8. The device of claim 1, further comprising a jaw base to which either or both the first (569, 115, 211) and second (568) jaws are pivotally coupled.

9. The device of claim 1, wherein the jaw input comprises a jaw pulley (119, 215), and the jaw mechanism (317) comprises a cam surface between the jaw pulley (119, 215) and the second jaw (568).

10. The device of claim 1, further comprising a releasable latching mechanism configured to hold the input lever (107, 201, 305, 549) locked in a closed position at the end of the input stroke.

## Patentansprüche

1. Medizinische Vorrichtung mit einer Backenanordnung (117, 213, 317), die durch ein Übertragungskabel (111, 207, 307, 566) betätigt wird, das in einer Übertragungsrichtung eine endliche Steifigkeit aufweist, in Biegung hochelastisch ist und in Zugrichtung steifer ist, um Kraft in der Übertragungsrichtung zu übertragen, wobei die Vorrichtung umfasst:
eine längliche Übertragungsführung (109', 209, 309), wobei das Übertragungskabel (111, 207, 307, 566) durch die Übertragungsführung (109', 209, 309) geführt ist;
eine Griffanordnung (103, 202, 303) an einem proximalen Ende der länglichen Übertragungsführung (109', 209, 309), wobei die Griffanordnung (103, 202, 303) einen Griffkörper (101, 203, 301, 501), einen Eingangshebel (107, 201, 305, 549), einen mit dem Übertragungskabel (111, 207, 307, 566) gekoppelten Griffausgang und einen Griffmechanismus (303) umfasst, der den Eingangshebel (107, 201, 305, 549) mit dem Griffausgang koppelt, wobei der Griffmechanismus (303) einen Eingangshub aufweist, der aus einer vollständigen Schließbewegung des Eingangshebels (107, 201, 305, 549) relativ zum Griffkörper (101, 203, 301, 501) besteht, wobei der Eingangshub ferner in einen ersten Teil und einen zweiten Teil unterteilt ist, wobei der erste Teil einem vollständigen Schließen der Backenanordnung (117, 213, 317) mit einer Verschiebung von 30 % bis 70 % der vollständigen Schließverschiebung des Eingangshebels (107, 201, 305, 549) entspricht und der zweite Teil der verbleibenden Verschiebung des Eingangshebels (107, 201, 305, 549) entspricht; und
wobei die Backenanordnung (117, 213, 317) distal zur länglichen Übertragungsführung (109', 209, 309) angeordnet ist, wobei die Backenanordnung (117, 213) eine erste Backe (569, 115, 211), eine zweite Backe (568), einen mit dem Übertragungskabel (111, 207, 307, 566) gekoppelten Backeneingangsantrieb und einen Backenmechanismus (317) aufweist, der den Backeneingangsantrieb mit der zweiten Backe (569, 115, 211) koppelt, wobei der Backenmechanismus (317) eine offene Konfiguration aufweist, wobei die erste (569, 115, 211) und die zweite (568) Backe relativ zueinander vollständig geöffnet sind, und eine geschlossene Konfiguration aufweist, wobei die erste (569, 115, 211) und die zweite (568) Backe vollständig geschlossen sind;
wobei ferner die Verschiebung des Eingangshebels (107, 201, 305, 549) relativ zum Griffkörper (101, 203, 301, 501), die dem ersten Teil des Eingangshubs entspricht, den Griffausgang betätigt, der wiederum den Backeneingangsantrieb über das Übertragungskabel (111, 207, 307, 566) betätigt, was wiederum die erste (569, 115, 211) und die zweite (568) Backe schließt, bis die erste (569, 115, 211) und die zweite (568) Backe einen Anschlag erreichen, und danach die Verschiebung des Eingangshebels (107, 201, 305, 549) relativ zum Griffkörper (101, 203, 301, 501), die dem zweiten Teil des Eingangshubs entspricht, das Übertragungskabel (111, 207, 307, 566) dehnt, wobei eine resultierende Spannung im Übertragungskabel (111, 207, 307, 566) durch eine Nockenfläche des Backenmechanismus (317) zu einer Haltekraft zwischen der ersten (569, 115, 211) und der zweiten (568) Backe verstärkt wird.

2. Vorrichtung nach Anspruch 1, wobei der Griffmechanismus (303) ein Gestänge oder eine Nocke umfasst.

3. Vorrichtung nach Anspruch 1, wobei der Griffmechanismus (303) ein Sechsgelenkgestänge umfasst.

4. Vorrichtung nach Anspruch 1, wobei die längliche Übertragungsführung (109', 209, 309) eine flexible Leitung oder eine längliche Welle oder beides umfasst.

5. Vorrichtung nach Anspruch 1, wobei das Übertragungskabel (111, 207, 307, 566) eine Steifigkeit in einer Übertragungsrichtung von weniger als 4,48 MPa (650 Pfund pro Zoll) aufweist.

6. Vorrichtung nach Anspruch 1, wobei der Griffmechanismus (303) so konfiguriert ist, dass er während des ersten Teils des Eingangshubs eine erste mechanische Kraftverstärkung und während des zweiten Teils des Eingangshubs eine zweite mechanische Kraftverstärkung bereitstellt, die größer ist als die erste mechanische Kraftverstärkung.

7. Vorrichtung nach Anspruch 1, wobei der Griffausgang eines oder mehrere der Folgenden umfasst: einen Schieber, eine Schubstange oder eine Zugstange.

8. Vorrichtung nach Anspruch 1, die ferner eine Backenbasis umfasst, an der entweder die erste (569, 115, 211) oder die zweite (568) Backe oder beide schwenkbar gekoppelt sind.

9. Vorrichtung nach Anspruch 1, wobei der Backeneingangsantrieb eine Backenrolle (119, 215) umfasst und der Backenmechanismus (317) eine Nockenfläche zwischen der Backenrolle (119, 215) und der zweiten Backe (568) umfasst.

10. Vorrichtung nach Anspruch 1, die ferner einen lösbaren Verriegelungsmechanismus umfasst, der so konfiguriert ist, dass er den Eingangshebel (107, 201, 305, 549) am Ende des Eingangshubs in einer geschlossenen Position verriegelt hält.

## Revendications

1. Dispositif médical ayant un ensemble de mâchoires (117, 213, 317) actionné par un câble de transmission (111, 207, 307, 566) ayant une rigidité finie dans une direction de transmission qui a une compliance en flexion élevée, et une plus grande rigidité en traction pour transmettre une force dans la direction de transmission, le dispositif comprenant :
un guide de transmission (109', 209, 309) allongé, dans lequel le câble de transmission (111, 207, 307, 566) est acheminé à travers le guide de transmission (109', 209, 309) ;
un ensemble poignée (103, 202, 303) à une extrémité proximale du guide de transmission (109', 209, 309) allongé, l'ensemble poignée (103, 202, 303) comprenant un corps de poignée (101, 203, 301, 501), un levier d'entrée (107, 201, 305, 549), une sortie de poignée accouplée au câble de transmission (111, 207, 307, 566), et un mécanisme de poignée (303) accouplant le levier d'entrée (107, 201, 305, 549) à la sortie de poignée, dans lequel le mécanisme de poignée (303) a une course d'entrée consistant en un déplacement de fermeture complète du levier d'entrée (107, 201, 305, 549) par rapport au corps de poignée (101, 203, 301, 501), en outre dans lequel la course d'entrée est divisée en une première partie et une deuxième partie, dans lequel la première partie correspond à une fermeture complète de l'ensemble de mâchoires (117, 213, 317) avec un déplacement de 30 % à 70 % du déplacement de fermeture complète du levier d'entrée (107, 201, 305, 549) et la deuxième partie correspond au déplacement restant du levier d'entrée (107, 201, 305, 549) ; et
dans lequel l'ensemble de mâchoires (117, 213, 317) est distal par rapport au guide de transmission (109', 209, 309) allongé, l'ensemble de mâchoires (117, 213) ayant une première mâchoire (569, 115, 211), une deuxième mâchoire (568), une entrée de mâchoire accouplée au câble de transmission (111, 207, 307, 566), et un mécanisme de mâchoire (317) accouplant l'entrée de mâchoire à la deuxième mâchoire (569, 115, 211), dans lequel le mécanisme de mâchoire (317) a une configuration ouverte lorsque les première (569, 115, 211) et deuxième (568) mâchoires sont complètement ouvertes l'une par rapport à l'autre et une configuration fermée lorsque les première (569, 115, 211) et deuxième (568) mâchoires sont complètement fermées ;
en outre dans lequel le déplacement du levier d'entrée (107, 201, 305, 549) par rapport au corps de poignée (101, 203, 301, 501) correspondant à la première partie de la course d'entrée actionne la sortie de la poignée qui à son tour actionne l'entrée de mâchoire par l'intermédiaire du câble de transmission (111, 207, 307, 566), ce qui à son tour ferme les première (569, 115, 211) et deuxième (568) mâchoires jusqu'à ce que les premières (569, 115, 211) et deuxième (568) mâchoires atteignent une butée, et ensuite le déplacement du levier d'entrée (107, 201, 305, 549) par rapport au corps de poignée (101, 203, 301, 501) correspondant à la deuxième partie de la course d'entrée étire le câble de transmission (111, 207, 307, 566), dans lequel une tension résultante dans le câble de transmission (111, 207, 307, 566) est amplifiée par une surface formant came du mécanisme de mâchoire (317) en une force de maintien entre les première (569, 115, 211) et deuxième (568) mâchoires.

2. Dispositif selon la revendication 1, dans lequel le mécanisme de poignée (303) comprend une bielle ou une came.

3. Dispositif selon la revendication 1, dans lequel le mécanisme de poignée (303) comprend une bielle à six barres.

4. Dispositif selon la revendication 1, dans lequel le guide de transmission (109', 209, 309) allongé comprend un conduit flexible et/ou un arbre allongé.

5. Dispositif selon la revendication 1, dans lequel le câble de transmission (111, 207, 307, 566) a une rigidité dans une direction de transmission inférieure à 4.48 MPa (650 livres par pouce).

6. Dispositif selon la revendication 1, dans lequel le mécanisme de poignée (303) est configuré pour fournir un premier avantage mécanique pendant la première partie de la course d'entrée et un deuxième avantage mécanique qui est supérieur au premier avantage mécanique pendant la deuxième partie de la course d'entrée.

7. Dispositif selon la revendication 1, dans lequel la sortie de poignée comprend un ou plusieurs parmi : une navette, une tige de poussée, ou une tige de traction.

8. Dispositif selon la revendication 1, comprenant en outre une base de mâchoire à laquelle les première (569, 115, 211) et/ou deuxième (568) mâchoires sont accouplées pivotantes.

9. Dispositif selon la revendication 1, dans lequel l'entrée de mâchoire comprend une poulie de mâchoire (119, 215), et le mécanisme de mâchoire (317) comprend une surface de came entre la poulie de mâchoire (119, 215) et la deuxième mâchoire (568).

10. Dispositif selon la revendication 1, comprenant en outre un mécanisme de blocage libérable configuré pour maintenir le levier d'entrée (107, 201, 305, 549) verrouillé en position fermée à la fin de la course d'entrée.
